# EUROPEAN PATENT SPECIFICATION

(11) **EP 3 408 289 B1**
(45) Date of publication and mention of the grant of the patent: **01.12.2021**
(21) Application number: 17701829.8
(22) Date of filing: 20.01.2017
(51) Int. Cl.: C07K 16/18, G01N 33/68

(54) **COLLAGEN TYPE VII ALPHA 1 ASSAY**
KOLLAGEN-TYP-VII-ALPHA-1-ASSAY
DOSAGE DU COLLAGÈNE DE TYPE VII ALPHA 1

(30) Priority: 28.01.2016 GB 201601571
(43) Date of publication of application: 05.12.2018
(73) Proprietor: Nordic Bioscience A/S, 2730 Herlev (DK)
(72) Inventor: SAND, Jannie, Marie, Bülow, 2760 Måløv (DK); LEEMING, Diana Julie, 2730 Herlev (DK); KARSDAL, Morten, 2100 København Ø (DK)
(74) Representative: Beck Greener LLP
(86) International application number: PCT/EP2017/051152
(87) International publication number: WO 2017/129480

(56) References cited:
- EP-A1- 2 479 190
- WO-A2-2010/115749
- K. A. ELSAID ET AL: "Association of articular cartilage degradation and loss of boundary-lubricating ability of synovial fluid following injury and inflammatory arthritis", ARTHRITIS & RHEUMATISM, vol. 52, no. 6, 1 June 2005 (2005-06-01), pages 1746-1755, XP055356235, US ISSN: 0004-3591, DOI: 10.1002/art.21038
- L. Y. SAKAI: "Type VII collagen is a major structural component of anchoring fibrils", THE JOURNAL OF CELL BIOLOGY : JCB, vol. 103, no. 4, 1 October 1986 (1986-10-01), pages 1577-1586, XP055356256, US ISSN: 0021-9525, DOI: 10.1083/jcb.103.4.1577
- A. RATTENHOLL ET AL: "Proteinases of the Bone Morphogenetic Protein-1 Family Convert Procollagen VII to Mature Anchoring Fibril Collagen", JOURNAL OF BIOLOGICAL CHEMISTRY, vol. 277, no. 29, 1 May 2002 (2002-05-01), pages 26372-26378, XP055356545, US ISSN: 0021-9258, DOI: 10.1074/jbc.M203247200

### SEQUENCE LISTING

<110> Nordic Bioscience A/S
<120> Collagen type VII alpha 1 Assay
<130> C7
<150> GB1601571.1
   <151> 2016-01-28
<160> 13
<170> BiSSAP 1.3.6
<210> 1
   <211> 10
   <212> PRT
   <213> Homo sapiens
<400> 1
   Gly Pro Pro Gly Pro Pro Gly Arg Leu Val
   1               5                   10
<210> 2
   <211> 6
   <212> PRT
   <213> Homo sapiens
<400> 2
   Pro Pro Gly Arg Leu Val
   1               5
<210> 3
   <211> 11
   <212> PRT
   <213> Artificial Sequence
<220>
   <223> C-terminal neo-epitope elongated
<220>
   <221> SITE
   <222> 11
   <223> X is one or more amino acids of the sequence of collagen type VII alpha 1
<400> 3
   Gly Pro Pro Gly Pro Pro Gly Arg Leu Val Xaa
   1               5                   10
<210> 4
   <211> 10
   <212> **PRT**
   <213> Homo sapiens
<400> 4
   Glu Ala Pro Arg Val Arg Ala Gln His Arg
   1               5                   10
<210> 5
   <211> 6
   <212> PRT
   <213> Homo sapiens
<400> 5
   Glu Ala Pro Arg Val Arg
   1               5
<210> 6
   <211> 11
   <212> PRT
   <213> Artificial Sequence
<220>
   <221> SITE
   <222> 1
   <223> X is one or more amino acids of the sequence of collagen type VII alpha 1
<220>
   <223> N-terminal neo-epitope elongated
<400> 6
   Xaa Glu Ala Pro Arg Val Arg Ala Gln His Arg
   1               5                   10
<210> 7
   <211> 10
   <212> PRT
   <213> Artificial Sequence
<220>
   <221> SITE
   <222> 1
   <223> Biotinylated with optional linker between biotin residue and peptide
<220>
   <223> C-terminal neo-epitope - biotinylated at N-terminus
<400> 7
   Gly Pro Pro Gly Pro Pro Gly Arg Leu Val
   1               5                   10
<210> 8
   <211> 10
   <212> PRT
   <213> Artificial Sequence
<220>
   <223> N-terminal neo-epitope - biotinylated at C-terminus
<220>
   <221> SITE
   <222> 10
   <223> Biotinylated with optional linker between biotin residue and peptide
<400> 8
   Glu Ala Pro Arg Val Arg Ala Gln His Arg
   1               5                   10
<210> 9
   <211> 13
   <212> PRT
   <213> Artificial Sequence
<220>
   <221> SITE
   <222> 1
   <223> KLH bound to CGG linker
<220>
   <223> immunisation peptide
<400> 9
   Cys Gly Gly Gly Pro Pro Gly Pro Pro Gly Arg Leu Val
   1               5                   10
<210> 10
   <211> 12
   <212> PRT
   <213> Artificial Sequence
<220>
   <221> SITE
   <222> 1
   <223> Biotinylated
<220>
   <223> Coater peptide
<400> 10
   Lys Lys Gly Pro Pro Gly Pro Pro Gly Arg Leu Val
   1               5                   10
<210> 11
   <211> 11
   <212> PRT
   <213> Artificial Sequence
<220>
   <223> elongated C-terminal sequence
<400> 11
   Gly Pro Pro Gly Pro Pro Gly Arg Leu Val Asp
   1 5 10
<210> 12
   <211> 13
   <212> PRT
   <213> Artificial Sequence
<220>
   <223> Immunisation peptide
<220>
   <221> SITE
   <222> 13
   <223> KLH bound
<400> 12
   Glu Ala Pro Arg Val Arg Ala Gln His Arg Gly Gly Cys
   1 5 10
<210> 13
   <211> 11
   <212> PRT
   <213> Artificial Sequence
<220>
   <223> Coater peptide
<220>
   <221> SITE
   <222> 11
   <223> Biotinylated
<400> 13 SEQUENCE LISTING
<110> Nordic Bioscience A/S
<120> Collagen type VII alpha 1 Assay
<130> C7
<150> GB1601571.1
   <151> 2016-01-28
<160> 13
<170> BiSSAP 1.3.6
<210> 1
   <211> 10
   <212> PRT
   <213> Homo sapiens
<400> 1
   Gly Pro Pro Gly Pro Pro Gly Arg Leu Val
   1 5 10
<210> 2
   <211> 6
   <212> PRT
   <213> Homo sapiens
<400> 2
   Pro Pro Gly Arg Leu Val
   1 5
<210> 3
   <211> 11
   <212> PRT
   <213> Artificial Sequence
<220>
   <223> C-terminal neo-epitope elongated
<220>
   <221> SITE
   <222> 11
   <223> X is one or more amino acids of the sequence of collagen type VII alpha 1
<400> 3
   Gly Pro Pro Gly Pro Pro Gly Arg Leu Val Xaa
   1 5 10
<210> 4
   <211> 10
   <212> PRT
   <213> Homo sapiens
<400> 4
   Glu Ala Pro Arg Val Arg Ala Gln His Arg
   1 5 10
<210> 5
   <211> 6
   <212> PRT
   <213> Homo sapiens
<400> 5
   Glu Ala Pro Arg Val Arg
   1 5
<210> 6
   <211> 11
   <212> PRT
   <213> Artificial Sequence
<220>
   <221> SITE
   <222> 1
   <223> X is one or more amino acids of the sequence of collagen type VII alpha 1
<220>
   <223> N-terminal neo-epitope elongated
<400> 6
   Xaa Glu Ala Pro Arg Val Arg Ala Gln His Arg
   1               5                   10
<210> 7
   <211> 10
   <212> PRT
   <213> Artificial Sequence
<220>
   <221> SITE
   <222> 1
   <223> Biotinylated with optional linker between biotin residue and peptide
<220>
   <223> C-terminal neo-epitope - biotinylated at N-terminus
<400> 7
   Gly Pro Pro Gly Pro Pro Gly Arg Leu Val
   1               5                   10
<210> 8
   <211> 10
   <212> PRT
   <213> Artificial Sequence
<220>
   <223> N-terminal neo-epitope - biotinylated at C-terminus
<220>
   <221> SITE
   <222> 10
   <223> Biotinylated with optional linker between biotin residue and peptide
<400> 8
   Glu Ala Pro Arg Val Arg Ala Gln His Arg
   1               5                   10
<210> 9
   <211> 13
   <212> PRT
   <213> Artificial Sequence
<220>
   <221> SITE
   <222> 1
   <223> KLH bound to CGG linker
<220>
   <223> immunisation peptide
<400> 9
   Cys Gly Gly Gly Pro Pro Gly Pro Pro Gly Arg Leu Val
   1 5 10
<210> 10
   <211> 12
   <212> PRT
   <213> Artificial Sequence
<220>
   <221> SITE
   <222> 1
   <223> Biotinylated
<220>
   <223> Coater peptide
<400> 10
   Lys Lys Gly Pro Pro Gly Pro Pro Gly Arg Leu Val
   1 5 10
<210> 11
   <211> 11
   <212> PRT
   <213> Artificial Sequence
<220>
   <223> elongated C-terminal sequence
<400> 11
   Gly Pro Pro Gly Pro Pro Gly Arg Leu Val Asp
   1 5 10
<210> 12
   <211> 13
   <212> PRT
   <213> Artificial Sequence
<220>
   <223> Immunisation peptide
<220>
   <221> SITE
   <222> 13
   <223> KLH bound
<400> 12
   Glu Ala Pro Arg Val Arg Ala Gln His Arg Gly Gly Cys
   1 5 10
<210> 13
   <211> 11
   <212> PRT
   <213> Artificial Sequence
<220>
   <223> Coater peptide
<220>
   <221> SITE
   <222> 11
   <223> Biotinylated
<400> 13
   Glu Ala Pro Arg Val Arg Ala Gln His Arg Lys
   1 5 10

## Description

### Technical Field

The present invention relates to antibodies which are reactive with fragments of collagen type VII alpha 1 comprising an N- or C-terminal neo-epitope, the use of said antibodies in an assay for detecting and quantifying said fragments of collagen type VII alpha 1, and the use of said assay for evaluating chronic obstructive pulmonary disease (COPD) or systemic sclerosis.

### Background Art

Collagen type VII is the main component of the anchoring fibrils that connects the basement membrane to the underlying interstitial matrix. It consists of three identical alpha-1 chains with two non-collagenous (NC) domains and a central collagenous triple helical domain. It has been identified in the basement membranes of skin and mucous membranes [1].

Collagen type VII has mainly been investigated for its role in dystrophic epidermolysis bullosa, a severe skin disease. Mutations in the collagen type VII alpha-1 chain leads to the formation of abnormal, diminished or absent anchoring fibrils which causes separation of epidermis from dermis and thus skin blistering [1]. Collagen type VII has also been identified as the protein at fault in epidermolysis bullosa acquisita, an autoimmune disease causing blistering of the skin and mucous membranes. It is caused by IgG autoantibodies directed at the collagen type VII NC1 domain [2].

Autoimmunity to collagen type VII has also been associated with inflammatory bowel disease and bullous systemic lupus erythematosus [3-4].

An up-regulation of collagen type VII level in the skin of patients suffering from systemic sclerosis has been identified [5]. Patients with systemic sclerosis have skin fibrosis and may present with fibrosis of internal organs including the lungs. One study has also identified a reduced level of collagen type VII protein in the anchoring fibrils in the airways in a monkey model of asthma [6].

In order to evaluate a pathogenic condition linked to collagen type VII it is necessary to produce assays capable of detecting and quantifying species related to the pathogenic condition.

Chen et al., Saleh et al. and Kim et al. independently developed ELISAs for detecting autoantibodies against the NC1 or NC2 domain of collagen type VII [7-9]. The methods comprise coating a microtiter plate with recombinant NC1 and/or NC2 domain of collagen type VII alpha-1, adding serum samples of interest, and using anti-human IgG antibody to detect autoantibodies present in the serum sample.

Recke et al. describes the generation of autoantibodies against the collagen type VII NC1 domain and investigated the pathogenic potential in human ex vivo models of epidermolysis bullosa acquisita [10]. They proposed the use of this as a diagnostic tool.

Sakai et al. raised a monoclonal antibody against collagen type VII [11]. The mAb was reactive only with intact collagen type VII.

Monoclonal and polyclonal antibodies targeting collagen type VII can be obtained commercially from several vendors.

WO2010/115749 teaches methods of diagnosing or quantifying fibrosis that comprise: conducting an immunoassay to measure neo-epitope containing protein fragments naturally present in a biofluid sample; and associating an elevation of said measure in said patient above a normal level with the presence or extent of fibrosis. Protein fragments naturally present in said sample are contacted with an immunological binding partner reactive with a neo-epitope formed by cleavage of a protein by a proteinase, and the extent of binding of peptide fragments to said immunological binding partner is measured, wherein said protein is collagen type III, collagen type I, collagen type IV, collagen type V, or collagen type VI, elastin, biglycan, decorin, lumican, versican, perlecan, neurocan, brevican, fibromodulin, serglycin, syndecan, betaglycan, vimentin, or C-reactive protein.

It has now been found that fragments of collagen type VII alpha 1 are detectable in circulation and could serve as potential biomarkers for evaluating pathological conditions linked to collagen type VII. Specifically, a link between collagen type VII alpha 1 fragments and the pathological conditions COPD and systemic sclerosis has been identified.

### Summary of the Invention

Accordingly, in a first aspect the present invention relates to an antibody reactive with a fragment of collagen type VII alpha 1 comprising an N- or C-terminal neo-epitope, wherein said antibody binds to the N- or C-terminal neo-epitope as defined in claim 1.

The antibody is a monoclonal antibody, and does not recognize or bind intact collagen type VII alpha 1.

In one embodiment, the monoclonal antibody reactive with a fragment of collagen type VII alpha 1 binds to the C-terminal neo-epitope, wherein the C-terminal neo-epitope is comprised in the amino acid sequence GPPGPPGRLV-COOH(SEQ ID NO: 1), and does not bind the elongated amino acid sequence GPPGPPGRLVX-COOH (SEQ ID NO: 3), wherein Xis one or more amino acids of the sequence of collagen type VII alpha 1.

Preferably, the antibody binds to a C-terminal neo-epitope comprising or consisting of the amino acid sequence PPGRLV-COOH (SEQ ID NO: 2). This C-terminal neo-epitope may be formed by cleavage of human collagen type VII alpha 1 at the Val-Asp bond between amino acids V1709-D1710 in the central collagenous domain of collagen type VII alpha 1.

<In another embodiment, the antibody binds to the N-terminal neo-epitope, wherein the N-terminal neo-epitope is comprised in the amino acid sequence H2N-EAPRVRAQHR (SEQID NO: 4), and does not bind to the elongated amino acid sequence H2N-XEAPRVRAQHR (SEQ ID NO: 6), wherein X is one or more amino acids of the sequence of collagen type VII alpha 1. Preferably, the monoclonal antibody binds to an N-terminal neo-epitope comprising the amino acid sequence H2N-EAPRVR (SEQ ID NO:5. This N-terminal neo-epitope may be formed by cleavage of human collagen type VII alpha 1 at the Ala-Glu bond between amino acids A16-E17 in the non-collagenous amino-terminal domain of collagen type VII alpha 1.

Antibodies described herein may be raised against a synthetic peptide corresponding to the N- or C-terminal neo-epitope amino acid sequence.

In a second aspect the present invention relates to a method of immunoassay for detecting in a biological sample a fragment of collagen type VII alpha 1 comprising an N- or C-terminal neo-epitope, said method comprising contacting said biological sample comprising said fragment of collagen type VII alpha 1 comprising said N- or C-terminal neo-epitope with an antibody as described herein, and determining the amount of binding of said antibody.

The method may be used to quantify the amount of the fragment of collagen type VII alpha 1 comprising said N- or C-terminal neo-epitope in biofluids. The biofluid may be, but is not limited to, serum, plasma, bronchoalveolar lavage fluid, sputum, saliva, or urine.

The immunoassay may be, but is not limited to, a competition assay or a sandwich assay. The immunoassay may be, but is not limited to, a radioimmunoassay or an enzyme-linked immunosorbent assay.

The method may further comprise the step of correlating the quantity of the fragment of collagen type VII alpha 1 comprising an N- or C-terminal neo-epitope determined by said method with standard collagen type VII related disease samples of known disease severity to evaluate the severity of a collagen type VII related disease.

Such collagen type VII related diseases may be, but are not limited to, chronic obstructive pulmonary disease (COPD) or systemic sclerosis.

It is envisaged that the method of the invention may be utilised in the quantitation, diagnosis and/or prognosis of such collagen type VII related diseases.

Described is a peptide, wherein the peptide has an N-terminal amino acid sequence corresponding to an amino acid sequence of an N-terminal neo-epitope of a fragment of collagen type VII alpha 1 comprising said N-terminal neo-epitope, or wherein the peptide has a C-terminal amino acid sequence corresponding to an amino acid sequence of an C-terminal neo-epitope of a fragment of collagen type VII alpha 1 comprising said C-terminal neo-epitope. Preferably, the peptide is ten amino acid residues in length, more preferably nine amino acid residues, more preferably eight amino acid residues, more preferably seven amino acid residues, and most preferably six amino acid residues in length. The peptide may be biotinylated.

Such peptide may have the amino acid sequence EAPRVRAQHR (SEQ ID NO: 4) or EAPRVR (SEQ ID NO: 5).

Alternatively, such peptide may have has the amino acid sequence GPPGPPGRLV (SEQ ID NO: 1) or PPGRLV (SEQ ID NO: 2).

In a third aspect the present invention relates to an assay kit for determining the quantity of a fragment of collagen type VII alpha 1 comprising an N- or C-terminal neo-epitope in a biological sample, the kit comprising an antibody as described herein and at least one of:
- a streptavidin coated 96 well plate
- a biotinylated peptide corresponding to the amino acid sequence of the N- or C-terminal neo-epitope, with an optional linker located between the biotin residue and the peptide
- a biotinylated secondary antibody for use in a sandwich immunoassay
- a calibrator peptide corresponding to the amino acid sequence of the N- or C-terminal neo-epitope
- an antibody HRP labeling kit
- an antibody radiolabeling kit
- an assay visualization kit

Preferably, the assay kit comprises a biotinylated peptide Biotin-L-GPPGPPGRLV (SEQ ID NO: 7), wherein L is an optional linker, and a calibrator peptide comprising the C-terminal sequence GPPGPPGRLV-COOH (SEQ ID NO: 1).

Preferably, the assay kit comprises a biotinylated peptide EAPRVRAQHR-L-Biotin (SEQ ID NO: 8), wherein L is an optional linker, and a calibrator peptide comprising the N-terminal sequence H₂N-EAPRVRAQHR (SEQ ID NO: 4).

### Definitions

The term "antibody" is used according to the invention in the broadest sense and specifically covers intact monoclonal antibodies, polyclonal antibodies, and antibody fragments, so long as they exhibit the desired biological activity.

"Antibody fragments" according to the invention comprise a portion of an intact antibody, preferably comprising the antigen-binding or variable region thereof. Examples of antibody fragments include Fab, Fab', F(ab')2, Fv and Fc fragments.

"A fragment of Collagen type VII alpha 1" according to the invention means a peptide fragment produced by protease cleavage of collagen type VII.

"N- or C-terminal neo-epitope" according to the invention means an N- or C-terminal epitope formed at a protease cleavage site of Collagen type VII alpha 1. For example, the following sequence of Collagen type VII alpha 1
...PGPPGPPGRLV ↓ DTGPGAREKGE...
would produce the N-terminal neo-epitope H₂N-DTGPGAREKGE... and the C-terminal neo-epitope ...PGPPGPPGRLV-COOH when cleaved by a protease at the site between the V¹⁷⁰⁹-D¹⁷¹⁰ peptide bond, as denoted by the symbol "↓".

"C7" as used herein refers to fragments of collagen type VII alpha 1 comprising the C-terminal neo-epitope GPPGPPGRLV-COOH (SEQ ID NO: 1).

"NB677" as used herein refers to fragments of collagen type VII alpha 1 comprising the N-terminal neo-epitope H₂N-EAPRVRAQHR (SEQ ID NO: 4).

### Figures

Figure 1 shows a calibration curve for the "C7" assay.
Figure 2 shows the correlation between "C7" and COPD.
Figure 3 shows the correlation between "C7" and systemic sclerosis.
Figure 4 shows a calibration curve for the "NB677" assay.
Figure 5. Clinical evaluation of serum C7 in systemic sclerosis. Serum C7 levels were assessed in healthy donors (n=70) and a cohort of patients with systemic sclerosis (SSc; n=119). Data are presented as Tukey's box plots. Statistical significance was evaluated by Mann-Whitney test. ***p<0.0001.

### Examples

### Example 1 - COPD Biomarker ("C7" Assay)

### Rationale

Mass spectrometry was performed on serum samples from a patient with COPD, a patient with idiopathic pulmonary fibrosis (IPF), and a healthy donor.

The initial mass spectrometry analyses identified peptides derived from collagen type VII in serum. Peptides were isolated from serum using IMAC Cu beads. Identity significance threshold for individual peptides were 51. Serum samples were analyzed using an orbitrap (OrbiB) instrument.

Fragments of collagen type VII alpha 1 comprising the C-terminal neo-epitope GPPGPPGRLV-COOH ("C7") (SEQ ID NO: 1) were found in the COPD sample but not in the IPF or healthy donor samples. The neo-epitope corresponds to the cleavage site located between amino acids Val-Asp at positions 1709-1710 of human collagen type VII. The protease responsible for this cleavage is as yet unknown. The sequence was analyzed using BLAST and was found to be unique for the collagen type VII alpha-1 chain.

### Antibody

A monoclonal antibody was raised against the C-terminal neo-epitope amino acid sequence GPPGPPGRLV-COOH (SEQ ID NO: 1).

Briefly, four to six-week-old Balb/C mice were immunized subcutaneously with 200 µL emulsified antigen and 50 µg of a C7 synthetic peptide (KLH-CGG-GPPGPPGRLV, SEQ ID NO: 9) using Freund's incomplete adjuvant. Immunizations were performed every 2^{nd} week until stable sera titer levels were reached. The mouse with highest serum titer was selected for fusion. The mouse was rested for one month and then boosted intravenously with 50 µg C7 peptide in 100 µL 0.9% sodium chloride solution three days before isolation of the spleen for cell fusion. Mouse spleen cells were fused with SP2/0 myeloma fusion partner cells. The resulting hybridoma cells were cloned using a semi-solid medium method, transferred into 96-well microtiter plates for further growth and incubated in a CO₂ incubator. Standard limited dilution was used to promote monoclonal growth.

### ELISA

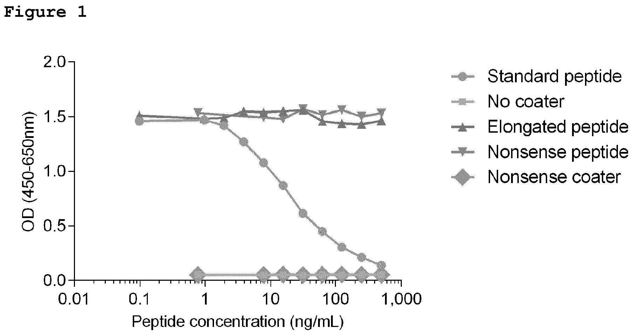

A competitive ELISA using the monoclonal antibody raised against C7 was performed using the following procedure:
Streptavidin-coated plates were coated with 100 µL/well of 2.5 ng/mL biotin-labeled peptide (Biotin-KKGPPGPPGRLV, SEQ ID NO: 10) diluted in assay buffer (50mM TBS-BTB, 2g/L NaCl, pH 8.0) and incubated at 20°C, 300 rpm shaking for 30 minutes. Plates were washed five times in washing buffer (20 nM TRIS, 50 mM NaCl, pH 7.2). Sample or standard peptide (20 µL) was added in double determinations and followed immediately by addition of 100 µL/well of 200 ng/mL HRP-labeled monoclonal antibody diluted in assay buffer and plates were incubated at 20°C, 300 rpm shaking for 3 hours. The standard peptide was a synthetic peptide (GPPGPPGRLV, SEQ ID NO: 1) with a starting concentration of 125 ng/mL and diluted 2-fold to create an 11 points calibration curve (Figure 1). After incubation, plates were washed five times in washing buffer. A volume of 100 µL 3,3',5,5'-tetramethylbenzidine (TMB) was added and incubated for 15 min at 20°C in the dark. To stop the enzyme reaction of TMB, 100 mL 0.1% sulphuric acid was added and the absorbance was measured at 450nm with 650nm as the reference using an ELISA reader. A calibration curve was plotted using a 4-parametric mathematical fit model. Each ELISA plate included both kit control and in-house quality control samples to monitor inter-assay variation. All samples were measured within the range of the assay. All samples below the lower limit of detection (LLOD) were assigned the value of LLOD.

The technical characteristics of the C7 ELISA are as follows:

| **Technical characteristics** | **Results** |
|---|---|
| **Biological matrix** | Human serum |
| | (undiluted measurements) |
| **Measurement range** | 1.5 - 105.6 ng/mL |
| **Normal range of healthy serum** | 3.5 ng/mL |
| **Inter-assay variation** | 13% (accepted if <15%) |
| **Intra-assay variation** | 9% (accepted if <10%) |
| **Dilution recovery** | Accepted |
| | (undiluted to 1 :8) |
| **Spiking recovery** | 166% (peptide in serum) |
| | 131% (serum in serum) |
| **Analyte stability** | Accepted |
| | (freeze/thaw and storage) |

The ELISA was shown to be specific for the cleavage site as reactivity was seen towards the standard peptide but not to an elongated peptide (GPPGPPGRLVD, SEQ ID NO: 11), indicating that the assay does not recognize intact collagen type VII protein (Figure 1).

### Clinical Utility

### COPD

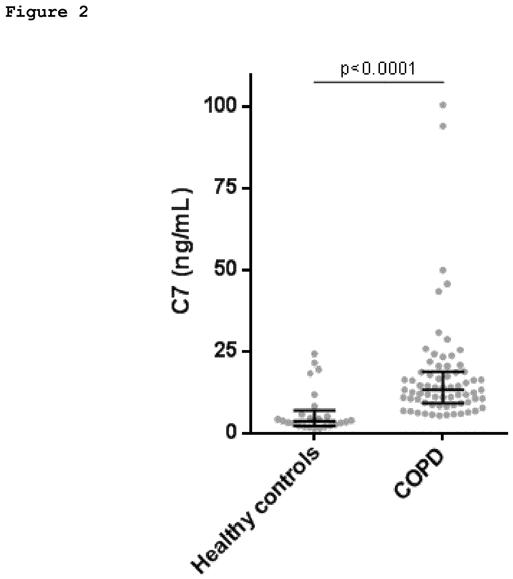

Serum levels of C7 were significantly elevated in a cohort of 68 patients with COPD when compared to healthy donors (Figure 2) .

These results show the utility of the C7 assay in identifying COPD, and may prove useful in evaluating COPD, for example as a diagnostic or prognostic tool.

### Systemic Sclerosis

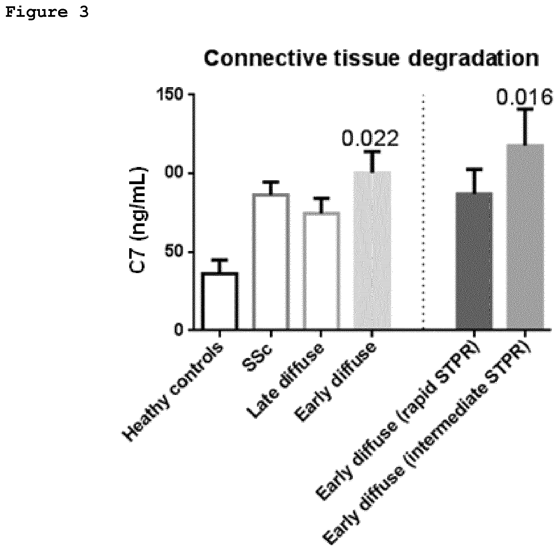

Serum levels of C7 were significantly elevated in 20 patients with early diffuse systemic sclerosis when compared to healthy control (p=0.022)(Figure 3). The early stage of systemic sclerosis is associated with high disease activity, whereas the late stage patients are progressing slowly. In the group of patients with early diffuse disease, a subpopulation with intermediate progression rate (defined by the skin thickness progression rate) had significantly elevated levels when compared to controls (p=0.016).

The elevated level of C7 in early stage systemic sclerosis when compared to late stage systemic sclerosis suggests that the C7 assay may be capable of differentiating between early and late stages of systemic sclerosis, thereby providing a potentially useful diagnostic and/or prognostic tool for evaluating systemic sclerosis.

### Example 2 ("NB677" Assay)

The signal peptide in collagen type VII alpha-1 is found at amino acids 1-16 [12]. The N-terminal neo-epitope sequence that is formed by cleavage of the signal peptide (17'.EAPRVRAQHR'26) was analyzed using BLAST and was found to be unique for the collagen type VII alpha-1 chain.

Following the success of the "C7" assay for COPD, it is postulated that this unique collagen type VII alpha-1 neo-epitope may also be useful in the identification and/or evaluation of COPD and/or systemic sclerosis.

### Antibody

Accordingly, a monoclonal antibody was raised against the N-terminal neo-epitope amino acid sequence H₂N-EAPRVRAQHR (SEQ ID NO: 4).

Briefly, four to six-week-old Balb/C mice were immunized subcutaneously with 200 µL emulsified antigen and 50 µg of a NB677 synthetic peptide (EAPRVRAQHR-GGC-KLH, SEQ ID NO: 12) using Freund's incomplete adjuvant. Immunizations were performed every 2^{nd} week until stable sera titer levels were reached. The mouse with highest serum titer was selected for fusion. The mouse was rested for one month and then boosted intravenously with 50 µg NB677 peptide in 100 µL 0.9% sodium chloride solution three days before isolation of the spleen for cell fusion. Mouse spleen cells were fused with SP2/0 myeloma fusion partner cells. The resulting hybridoma cells were cloned using a semi-solid medium method, transferred into 96-well microtiter plates for further growth and incubated in a CO₂ incubator. Standard limited dilution was used to promote monoclonal growth.

### ELISA

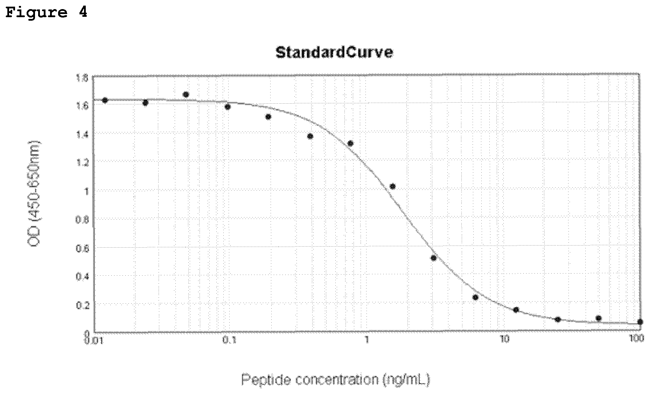

A competitive ELISA using the monoclonal antibody was performed using the following procedure:
Streptavidin-coated plates were coated with 100 µL/well of 2.0 ng/mL biotin-labeled peptide (EAPRVRAQHR-Lys-Biotin, SEQ ID NO: 13) diluted in coating buffer (50 mM PBS-BTE, 8g/L NaCl, 10% sorbitol) and incubated at 20°C, 300 rpm shaking for 30 minutes. Plates were washed five times in washing buffer (20 nM TRIS, 50 mM NaCl, pH 7.2). Standard peptide (20 µL) was added in double determinations and followed immediately by addition of 100 µL/well of 120 ng/mL monoclonal antibody diluted in assay buffer (25 mM PBS-BTB, 8g/L NaCl) and plates were incubated at 4°C, 300 rpm shaking for 20 hours. The standard peptide was a synthetic peptide (EAPRVRAQHR, SEQ ID NO: 4) with a starting concentration of 100 ng/mL and diluted 2-fold to create a calibration curve (Figure 4). After incubation, plates were washed five times in washing buffer. 100 µL/well of secondary HRP-labeled antibody (rabbit anti-mouse IgG) was added diluted 1:3000 in assay buffer and plates were incubated at 20°C, 300 rpm shaking for 1 hour. A volume of 100 µL 3,3',5,5'-tetramethylbenzidine (TMB) was added and incubated for 15 min at 20°C in the dark. To stop the enzyme reaction of TMB, 100 mL 0.1% sulphuric acid was added and the absorbance was measured at 450nm with 650nm as the reference using an ELISA reader. A calibration curve was plotted using a 4-parametric mathematical fit model. The monoclonal antibody directed to the collagen type VII alpha 1 N-terminal neo-epitope has been confirmed to recognize the desired sequence, assessed by the reactivity to the standard peptide.

### Example 3

The C7 ELISA was evaluated in a second, larger cohort of patients with systemic sclerosis (SSc). The C7 ELISA was recalibrated (compared to the previous example) to improve accuracy of the assessments in human serum.

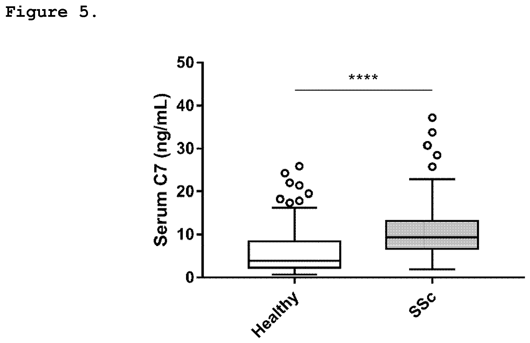

**Results:** The biological relevance of the C7 ELISA was evaluated by comparing serum levels in healthy donors (n=70) with patients with SSc (n=119). Data are shown in Figure 5. Median serum C7 level was significantly elevated in patients with SSc (9.3 ng/mL [IQR 6.7-13.2]) as compared with healthy donors (3.9 ng/mL [IQR 2.3-8.3 ng/mL]; p<0.0001).

The clinical data support that serum C7 levels are elevated in patients with SSc

In conclusion, the novel assays described herein utilise antibodies specific for an N- or C-terminal neo-epitope of collagen VII alpha 1. To the best of our knowledge, this is the first time that collagen type VII has been associated with COPD. Accordingly, it is envisaged that these assays may be used for assessing COPD as well as systemic sclerosis.

In this specification, unless expressly otherwise indicated, the word 'or' is used in the sense of an operator that returns a true value when either or both of the stated conditions is met, as opposed to the operator 'exclusive or' which requires that only one of the conditions is met. The word 'comprising' is used in the sense of 'including' rather than in to mean 'consisting of'.

### References

[1] Chung, H. J. and J. Uitto. 2010. Type VII collagen: the anchoring fibril protein at fault in dystrophic epidermolysis bullosa. Dermatol.Clin. 28:93-105.
[2] Chen, M., G. H. Kim, L. Prakash, and D. T. Woodley. 2012. Epidermolysis bullosa acquisita: autoimmunity to anchoring fibril collagen. Autoimmunity 45:91-101.
[3] Hundorfean, G., M. F. Neurath, and C. Sitaru. 2010. Autoimmunity against type VII collagen in inflammatory bowel disease. J.Cell Mol.Med. 14:2393-2403.
[4] Gammon, W. R., D. T. Woodley, K. C. Dole, and R. A. Briggaman. 1985. Evidence that anti-basement membrane zone antibodies in bullous eruption of systemic lupus erythematosus recognize epidermolysis bullosa acquisita autoantigen. J.Invest Dermatol. 84:472-476.
[5] Rudnicka, L., J. Varga, A. M. Christiano, R. V. Iozzo, S. A. Jimenez, and J. Uitto. 1994. Elevated expression of type VII collagen in the skin of patients with systemic sclerosis. Regulation by transforming growth factor-beta. J.Clin.Invest 93:1709-1715.
[6] Evans, M. J., M. V. Fanucchi, L. A. Miller, M. A. Carlson, S. J. Nishio, and D. M. Hyde. 2010. Reduction of collagen VII anchoring fibrils in the airway basement membrane zone of infant rhesus monkeys exposed to house dust mite. Am.J.Physiol Lung Cell Mol.Physiol 298:L543-L547.
[7] Chen, M., L. S. Chan, X. Cai, E. A. O'Toole, J. C. Sample, and D. T. Woodley. 1997. Development of an ELISA for rapid detection of anti-type VII collagen autoantibodies in epidermolysis bullosa acquisita. J.Invest Dermatol. 108:68-72.
[8] Saleh, M. A., K. Ishii, Y. J. Kim, A. Murakami, N. Ishii, T. Hashimoto, E. Schmidt, D. Zillikens, Y. Shirakata, K. Hashimoto, et al. 2011. Development of NC1 and NC2 domains of type VII collagen ELISA for the diagnosis and analysis of the time course of epidermolysis bullosa acquisita patients. J.Dermatol.Sci 62:169-175.
[9] Kim, J. H., Y. H. Kim, S. Kim, E. B. Noh, S. E. Kim, A. Vorobyev, E. Schmidt, D. Zillikens, and S. C. Kim. 2013. Serum levels of anti-type VII collagen antibodies detected by enzyme-linked immunosorbent assay in patients with epidermolysis bullosa acquisita are correlated with the severity of skin lesions. J.Eur.Acad Dermatol.Venereol. 27:e224-e230.
[10] Recke, A., C. Sitaru, G. Vidarsson, M. Evensen, M. T. Chiriac, R. J. Ludwig, and D. Zillikens. 2010. Pathogenicity of IgG subclass autoantibodies to type VII collagen: induction of dermal-epidermal separation. J.Autoimmun. 34:435-444.
[11] Sakai, L. Y., D. R. Keene, N. P. Morris, and R. E. Burgeson. 1986. Type VII collagen is a major structural component of anchoring fibrils. J.Cell Biol. 103:1577-1586.
[12] Uniprot. http://www.uniprot.org/uniprot/Q02388. Accessed 2015.

## Claims

1. A monoclonal antibody reactive with a fragment of collagen type VII alpha 1 comprising an N- or C-terminal neo-epitope formed by protease cleavage of Collagen type VII alpha 1:
wherein said monoclonal antibody binds to the N-terminal neo-epitope, wherein the N-terminal neo-epitope is comprised in the amino acid sequence H₂N-EAPRVRAQHR (SEQ ID NO: 4), and does not bind to the elongated amino acid sequence H₂N-XEAPRVRAQHR (SEQ ID NO: 6), wherein X is one or more amino acids of the sequence of collagen type VII alpha 1; or
wherein said monoclonal antibody binds to the C-terminal neo-epitope, wherein the C-terminal neo-epitope is comprised in the amino acid sequence GPPGPPGRLV-COOH (SEQ ID NO: 1), and does not bind the elongated amino acid sequence GPPGPPGRLVX-COOH (SEQ ID NO: 3), wherein X is one or more amino acids of the sequence of collagen type VII alpha 1.

2. A monoclonal antibody as claimed in claim 1, wherein said antibody binds to a C-terminal neo-epitope comprising the amino acid sequence PPGRLV-COOH (SEQ ID NO: 2).

3. A monoclonal antibody as claimed in claim 1, wherein said antibody binds to an N-terminal neo-epitope comprising the amino acid sequence H₂N-EAPRVR (SEQ ID NO: 5) .

4. A method of immunoassay for detecting in a biological sample a fragment of collagen type VII alpha 1 comprising an N- or C-terminal neo-epitope, said method comprising contacting said biological sample comprising said fragment of collagen type VII alpha 1 comprising said N- or C-terminal neo-epitope with a monoclonal antibody as claimed in any preceding claim, and determining the amount of binding of said antibody.

5. A method as claimed in claim 4, wherein said method is used to quantify the amount of the fragment of collagen type VII alpha 1 comprising said N- or C-terminal neo-epitope in biofluids.

6. A method as claimed in claim 5, wherein said biofluid is serum, plasma, bronchoalveolar lavage fluid, sputum, or urine.

7. A method as claimed in claim 4, wherein said immunoassay is a competition assay or a sandwich assay.

8. A method as claimed in claim 4, wherein said immunoassay is a radioimmunoassay or an enzyme-linked immunosorbent assay.

9. A method as claimed in claim 5, further comprising correlating the quantity of the fragment of collagen type VII alpha 1 comprising an N- or C-terminal neo-epitope determined by said method with standard collagen type VII related disease samples of known disease severity to evaluate the severity of a collagen type VII related disease wherein the collagen type VII related disease is chronic obstructive pulmonary disease (COPD) or systemic sclerosis.

10. An assay kit for determining the quantity of a fragment of collagen type VII alpha 1 comprising an N- or C-terminal neo-epitope in a biological sample, the kit comprising an antibody as claimed in any of claims 1 to 3 and at least one of:
- a streptavidin coated 96 well plate
- a biotinylated peptide corresponding to the amino acid sequence of the N- or C-terminal neo-epitope, with an optional linker located between the biotin residue and the peptide
- a biotinylated secondary antibody suitable for use in a
sandwich immunoassay
- a calibrator peptide corresponding to the amino acid sequence of the N- or C-terminal neo-epitope
- an antibody HRP labeling kit
- an antibody radiolabeling kit
- an assay visualization kit

11. An assay kit as claimed in claim 10, comprising a biotinylated peptide Biotin-L-GPPGPPGRLV (SEQ ID NO: 7), wherein L is an optional linker, and a calibrator peptide comprising the C-terminal sequence GPPGPPGRLV-COOH (SEQ ID NO: 1); or
comprising a biotinylated peptide EAPRVRAQHR-L-Biotin (SEQ ID NO: 8), wherein L is an optional linker, and a calibrator peptide comprising the N-terminal sequence H₂N-EAPRVRAQHR (SEQ ID NO: 4).

## Patentansprüche

1. Monoklonaler Antikörper, der mit einem Fragment von Kollagen Typ VII alpha 1 reagiert, das ein N- oder C-terminales Neo-Epitop umfasst, das durch Proteasespaltung von Kollagen Typ VII alpha 1 ausgebildet wird:
wobei der monoklonale Antikörper an das N-terminale Neo-Epitop bindet, wobei das N-terminale Neo-Epitop in der Aminosäuresequenz H₂N-EAPRVRAQHR (SEQ ID NO: 4) enthalten ist, und nicht an die längliche Aminosäuresequenz H₂N-XEAPRVRAQHR (SEQ ID NO: 6) bindet, wobei X eine oder mehrere Aminosäuren der Sequenz von Kollagen Typ VII alpha 1 ist; oder
wobei der monoklonale Antikörper an das C-terminale Neo-Epitop bindet, wobei das C-terminale Neo-Epitop in der Aminosäuresequenz GPPGPPGRLV-COOH (SEQ ID NO: 1) enthalten ist, und nicht an die längliche Aminosäuresequenz GPPGPPGRLVX-COOH (SEQ ID NO: 3) bindet, wobei X eine oder mehrere Aminosäuren der Sequenz von Kollagen Typ VII alpha 1 ist.

2. Monoklonaler Antikörper nach Anspruch 1, wobei der Antikörper an ein C-terminales Neo-Epitop bindet, das die Aminosäuresequenz PPGRLV-COOH (SEQ ID NO: 2) umfasst.

3. Monoklonaler Antikörper nach Anspruch 1, wobei der Antikörper an ein N-terminales Neo-Epitop bindet, das die Aminosäuresequenz H₂N-EAPRVR (SEQ ID NO: 5) umfasst.

4. Immunoassay-Verfahren zum Nachweisen eines Fragments von Kollagen Typ VII alpha 1 in einer biologischen Probe, das ein N- oder C-terminales Neo-Epitop umfasst, wobei das Verfahren ein Inberührungbringen der biologischen Probe, die das Fragment von Kollagen Typ VII alpha 1 umfasst, das das N- oder C-terminale Neo-Epitop umfasst, mit einem monoklonalen Antikörper nach einem der vorhergehenden Ansprüche und ein Bestimmen der Bindungsmenge des Antikörpers umfasst.

5. Verfahren nach Anspruch 4, wobei das Verfahren verwendet wird, um die Menge des Fragments von Kollagen Typ VII alpha 1, das das N- oder C-terminale Neo-Epitop umfasst, in Biofluiden zu quantifizieren.

6. Verfahren nach Anspruch 5, wobei das Biofluid ein Serum, ein Plasma, ein Bronchoalveoläre-Lavage-Fluid, Sputum oder Urin ist.

7. Verfahren nach Anspruch 4, wobei der Immunoassay ein kompetitiver Assay oder ein Sandwich-Assay ist.

8. Verfahren nach Anspruch 4, wobei der Immunoassay ein Radioimmunoassay oder ein Enzymimmunoassay ist.

9. Verfahren nach Anspruch 5, das ferner ein Korrelieren der Menge des Fragments von Kollagen Typ VII alpha 1, das ein N- oder C-terminales Neo-Epitop umfasst, das durch das Verfahren bestimmt wird, mit Standardkollagen-Typ-VII-bezogenen Erkrankungsproben bekannter Erkrankungsschwere umfasst, um die Schwere einer Kollagen-Typ-VII-bezogenen Erkrankung zu bewerten, wobei die Kollagen-Typ-VIIbezogene Erkrankung chronisch obstruktive Lungenerkrankung (chronic obstructive pulmonary disease - COPD) oder systemische Sklerose ist.

10. Assay-Kit zum Bestimmen der Menge eines Fragments von Kollagen Typ VII alpha 1, das ein N- oder C-terminales Neo-Epitop umfasst, in einer biologischen Probe, wobei das Kit einen Antikörper nach einem der Ansprüche 1 bis 3 und Folgendes umfasst:
- eine mit Streptavidin beschichtete 96-Well-Platte
- ein biotinyliertes Peptid, das der Aminosäuresequenz des N- oder C-terminalen Neo-Epitops entspricht, mit einem optionalen Linker, der sich zwischen dem Biotinrest und dem Peptid befindet
- einen biotinylierten sekundären Antikörper, der zur Verwendung in einem Sandwich-Immunoassay geeignet ist
- ein Kalibratorpeptid, das der Aminosäuresequenz des N- oder C-terminalen Neo-Epitops entspricht
- ein Antikörper-HRP-Markierungs-Kit
- ein Antikörper-Radiomarkierungs-Kit und/oder
- ein Assay-Visualisierungs-Kit

11. Assay-Kit nach Anspruch 10, das ein biotinyliertes Peptid Biotin-L-GPPGPPGRLV (SEQ ID NO: 7), wobei L ein optionaler Linker ist, und ein Kalibratorpeptid umfasst, das die C-terminale Sequenz GPPGPPGRLV-COOH (SEQ ID NO: 1) umfasst; oder
ein biotinyliertes Peptid EAPRVRAQHR-L-Biotin (SEQ ID NO: 8), wobei L ein optionaler Linker ist, und ein Kalibratorpeptid umfasst, das die N-terminale Sequenz H₂N-EAPRVRAQHR (SEQ ID NO: 4) umfasst.

## Revendications

1. Anticorps monoclonal réactif avec un fragment de collagène de type VII alpha 1 comprenant un néo-épitope N- ou C-terminal formé par clivage de protéase de Collagène de type VII alpha 1 :
ledit anticorps monoclonal se liant au néo-épitope N-terminal, le néo-épitope N-terminal étant compris dans la séquence d'acides aminés H2N-EAPRVRAQHR (SEQ ID NO : 4) et ne se liant pas à la séquence d'acides aminés allongée H₂N-XEAPRVRAQHR (SEQ ID NO : 6), X représentant un ou plusieurs acides aminés de la séquence du collagène de type VII alpha 1 ; ou
ledit anticorps monoclonal se liant au néo-épitope C-terminal, le néo-épitope C-terminal étant compris dans la séquence d'acides aminés GPPGPPGRLV-COOH (SEQ ID NO : 1) et ne se liant pas à la séquence d'acides aminés allongée GPPGPPGRLVX-COOH (SEQ ID NO : 3), X représentant un ou plusieurs acides aminés de la séquence du collagène de type VII alpha 1.

2. Anticorps monoclonal selon la revendication 1, ledit anticorps se liant à un néo-épitope C-terminal comprenant la séquence d'acides aminés PPGRLV-COOH (SEQ ID NO : 2).

3. Anticorps monoclonal selon la revendication 1, ledit anticorps se liant à un néo-épitope N-terminal comprenant la séquence d'acides aminés H₂N-EAPRVR (SEQ ID NO : 5).

4. Procédé d'immunoessai permettant de détecter dans un échantillon biologique un fragment de collagène de type VII alpha 1 comprenant un néo-épitope N- ou C-terminal, ledit procédé consistant à mettre en contact ledit échantillon biologique comprenant ledit fragment de collagène de type VII alpha 1 comprenant ledit Néo-épitope N- ou C-terminal avec un anticorps monoclonal selon l'une quelconque des revendications précédentes et détermination de la quantité de liaison dudit anticorps.

5. Procédé selon la revendication 4, ledit procédé étant utilisé pour quantifier la quantité du fragment de collagène de type VII alpha 1 comprenant ledit néo-épitope N- ou C-terminal dans les biofluides.

6. Procédé selon la revendication 5, ledit biofluide étant du sérum, du plasma, du fluide de lavage broncho-alvéolaire, des expectorations ou de l'urine.

7. Procédé selon la revendication 4, ledit immunodosage étant un dosage par compétition ou un dosage en sandwich.

8. Procédé selon la revendication 4, ledit immunodosage étant un dosage radio-immunologique ou un dosage immunosorbant lié à une enzyme.

9. Procédé selon la revendication 5, comprenant en outre la corrélation de la quantité du fragment de collagène de type VII alpha 1 comprenant un néo-épitope N- ou C-terminal déterminé par ledit procédé avec des échantillons de maladie liée au collagène de type VII standard d'une maladie connue la gravité pour évaluer la gravité d'une maladie liée au collagène de type VII, la maladie liée au collagène de type VII étant la maladie pulmonaire obstructive chronique (MPOC) ou la sclérose systémique.

10. Kit de dosage pour déterminer la quantité d'un fragment de collagène de type VII alpha 1 comprenant un néo-épitope N- ou C-terminal dans un échantillon biologique, le kit comprenant un anticorps selon l'une quelconque des revendications 1 à 3 et :
- une plaque à 96 puits revêtue de streptavidine et/ou
- un peptide biotinylé correspondant à la séquence d'acides aminés du néo-épitope N- ou C-terminal, avec un lieur éventuel situé entre le résidu de biotine et le peptide et/ou
- un anticorps secondaire biotinylé approprié pour être utilisé dans un immunodosage en sandwich et/ou
- un peptide étalon correspondant à la séquence d'acides aminés du néo-épitope N- ou C-terminal et/ou
- un kit de marquage d'anticorps HRP et/ou
- un kit de radiomarquage d'anticorps et/ou
- un kit de visualisation de dosage

11. Kit de dosage selon la revendication 10, comprenant un peptide biotinylé Biotin-L-GPPGPPGRLV (SEQ ID NO : 7), L représentant un lieur éventuel, et un peptide étalon comprenant la séquence C-terminale GPPGPPGRLV-COOH (SEQ ID NO : 1) ; ou comprenant un peptide biotinylé EAPRVRAQHR-L-Biotin (SEQ ID NO : 8), L représentant un lieur éventuel, et un peptide étalon comprenant la séquence N-terminale H₂N-EAPRVRAQHR (SEQ ID NO : 4).
